# EUROPEAN PATENT SPECIFICATION

(11) **EP 1 051 963 B1**
(45) Date of publication and mention of the grant of the patent: **02.08.2006**
(21) Application number: 00109584.3
(22) Date of filing: 05.05.2000
(51) Int. Cl.: A61Q 17/04, A61K 8/35, A61K 8/37, A61K 8/81

(54) **Photostable cosmetic light screening compositions**
Lichtstabile kosmetische Sonnenschutzmittel
Composition cosmétique d'écran solaire photostable

(30) Priority: 12.05.1999 EP 99109515
(43) Date of publication of application: 15.11.2000
(73) Proprietor: DSM IP Assets B.V., 6411 TE Heerlen (NL)
(72) Inventor: Berset, Guy, 1213 Onex (CH); Pittet, Gilbert, 1296 Coppet (CH); Richard, Joel François, 49160 Longué (FR); Sidrac, Dominique, 74160 St Julien (en Genevois) (FR)
(74) Representative: Steck, Melanie

(56) References cited:
- EP-A- 0 573 229
- EP-A- 0 610 026
- EP-A- 0 669 124
- EP-A- 0 780 119
- DE-A- 4 336 407
- FR-A- 2 658 075
- FR-A- 2 681 248
- FR-A- 2 687 914
- FR-A- 2 755 856
- US-A- 5 876 699

## Description

The invention relates to photostable cosmetic or pharmaceutical light screening compositions for the protection of the human epidermis or hair against ultraviolet rays of wavelengths between 280 and 400 nm (UV-A/UV-B).

More specifically, the invention relates to photostable light screening compositions containing a dibenzoyl methane UV-A screening agent and a p-methoxycinnamate UV-B screening agent wherein one of the screening agents is incorporated into a polymer matrix.

EP-A 0 875 240 discloses the use of a composition comprising cyclodextrins (I) and cosmetically or dermatologically harmless dibenzoylmethane derivatives (II) and/or cinnamic acid derivatives (III), in cosmetic and dermatological light protection formulations (e.g. skin care formulations). (I) acts hereby as a UV stabiliser for (II) and (III), many of which are not photostable.

DE-OS 37 41 420 discloses a light-stable cosmetic for protecting the human epidermis against UV radiation containing (a) a cosmetically acceptable carrier with a fat phase, (b) 1-3 weight% of 4-(1,1-dimethylethyl)-4'-methoxydibenzoylmethane and (c) at least 4.5% of p-methylbenzylidene camphor, in the ratio by weight of (c):(b) of at least 3. The ratio by weight of (c):(b) is at least 6. Component (b) is hereby stabilised against UV radiation by the addition of (c). The cosmetic is an oily or oil/alcoholic lotion, a fatty or oil/alcoholic gel, a solid rod, or an emulsion, or is packed as an aerosol, and may contain thickeners, softeners, moisturisers, surfactants, preservatives, antifoams, perfumes, oils, waxes, low mono- or poly-alcohols, propellants, dyes and pigments. Emulsions, as creams or milks, may contain fatty alcohols, esters of fatty acids (especially triglycerides), fatty acids, lanolin, natural or synthetic oils or waxes, and emulsifiers in presence of water. Oily lotions may contain fatty acid esters and natural or synthetic oils and/or waxes. Oil/alcoholic lotions may contain oils, waxes, fatty acid esters (especially triglycerides), and lower alcohols, glycols and/or polyols. Oily/alcoholic gels may contain, in addition to an oil, natural or synthetic waxes, a lower alcohol or polyol, and a thickener.

EP-A 0 669 124 describes compositions comprising UV radiation absorbing agent(s) and 0.1-50 weight% (based on the total weight of nonvolatiles) of latex particles of a particle size of 50-500 nm. The addition of the latex particles improves the UV radiation absorption of the compositions. The compositions are e.g. used on human skin and hair, such as in the form of cosmetics, sunscreens and hair care products. The compositions of EP-A 0 669 124 are also useful for improving the UV absorption and protection for coatings on plant life, plastics, wood, etc.

Skin is protected against UV radiation according to FR-A 2 687 914 using a combination of a non-encapsulated anti-UV component (I) and an encapsulated anti-UV component (II), such that inactivation of (I) by contact with air is compensated by release of (II) from its encapsulated form. This method provides sustained protection. Also disclosed is a cosmetic composition for protecting the skin against UV radiation and permitting tanning without erythema, comprising non-encapsulated (I), encapsulated (II) and an excipient, where (I) and (II) comprise organic UV-A and/or UV-B absorbers. (I) and (II) comprise organic UV-A and/or UV-B absorbers. (II) is in the form of microspheres with an average diameter of 50-900 (especially 100-800 nm), coated with a polymeric material, especially a combination of collagen and chondroitin sulphate. (II) may also include a radical scavenger and/or antioxidant.

DE-OS 43 36 407 discloses cosmetic compositions comprising: (a) a solid, particulate water-insoluble matrix of at least one water-insoluble, non-polymeric, matrix-forming material which is solid at room temperature; and (b) a water-insoluble cosmetic active agent embedded in the matrix. The matrix particles have an average size of up to 1 mu, as well as aqueous dispersion comprising such compositions. The cosmetic compositions are useful, e.g. in sunscreens, nail varnishes, hair care products, etc. Advantageously the compositions may be prepared without the use of polymerisation processes or organic solvents which must later be removed. They may contain a large variety of cosmetic active agents and may allow delayed release of active agent.

The stable O/W emulsion according to EP-A 0 356 196 contains 2-hydroxyethyl methacrylate homopolymer (PHEMA) from which a non-gummy, hygroscopic, flexible and pliable film can be deposited on an epidermal surface as a cosmetic. The emulsion is manufactured in a multi-step sequence comprising the following steps: (i) separate formation of oil and water phases; (ii) combining the oil phase with a water phase into either an O/W or W/O emulsion; (iii) providing PHEMA in solution in combination with a plasticiser; (iv) providing the solution in an aqueous phase; (v) provinding an emulsifying agent in the desired O/W emulsion as part of an aqueous phase or to another of the aqueous phases which will ultimately be combined in forming the desired O/W emulsion; and (vi) combining the various phases to make the O/W emulsion. PHEMA can be used, optionally together with a sunscreening agent to form a smooth cosmetic skin lotion, which is free from greasiness, moisture-resistant, and with non-gummy rubout characteristics. The emulsions containing e.g. ammonium stearate as emulsifiers are stable to heating at 40°C for over 6 months.

EP-A 0 265 228 discloses a skin protecting agent containing copolymers of ethyl acrylate (EA) and methacrylic acid (MMA) in a weight ratio of 75:25 to 95:5 comprising a maximum amount of 50 ppm of residual monomer and substantially no surfactant. In the preparation of such copolymers the copolymerisation of the 75 to 95 parts by weight of EA and 25 to 5 parts by weight of MAA is effected preferably in deionised water in the presence of an initiator, e.g. persulphate. The polymerisation temperature is maintained to decrease the residual monomer concentration. The amount of persulphate is 0.2 to 4, preferably 0.4 to 3 phm. The polymerisation temperature is 45 to 98°C, preferably 65 to 95°C. The post-treatment time may be reduced by increasing the temperature or by adding further persulphate or a redox catalyst. The copolymers are useful cosmetically or protectively, are less irritating because of the absence of surfactants, and form a thin film on the skin which is resistant to acidic or neutral environments but is easily dissolved in slightly alkaline media, e.g. toilet soap and water, and thus readily removed by washing.

The waterproof sunscreen composition of US 4,699,779 comprises: (A) 15 to 95 weight% of water; (B) 0.1 to 6.0 weight% of an ethyl cellulose polymer having an average ethoxyl substitution of 2.25 to 2.58 per anhydroglucose unit (AGU) which equates to an ethoxyl content of 45 to 52%; (C) 1.0 to 30.0 weight% of an UV absorber; (D) 0.01 to 12 weight% of a surfactant; and (E) 0.03 to 5.0 weight% of an alkaline dispersion promoting agent. Preferred UV absorbers are e.g. p-aminobenzoic acid, 2-ethoxyethyl p-methoxycimamate, diethanolamine p-methoxycinnamate, digalloyl trioleate, 2,2'-dihydroxy -4-methoxybenzophenone, etc. Preferred dispersion promoter are C₃₋₆ alkylamines, C₃₋₆ alkanolamines and ammonium hydroxide. Preferred surfactants are nonionic esters, nonionic ethers, sodium alkylbenzene sulphonates etc. and alkali-fatty acid soaps, e.g. ammonium myristate, potassium oleate or triethamolamine stearate. Useful ethyl cellulose polymers are available as dispersions, i.e. XD-30543.40 and XD-30543.30 from Dow Chemical Co. Preferred thereof is "Ethocel". The composition of US 4,699,779 is solvent-free, is able to suspend insoluble particulate material, can be applied to the skin evenly and easily, dries quickly without leaving the skin feeling tacky, and is resistant to removal by water and perspiration, and gives effective protection against the harmful effects of the sun's radiation.

The composition according to US 4,671,955 comprises: (A) 0.5 to 20 weight% of an organo-soluble, water-insoluble ethyl hydroxyethyl cellulose polymer having a (D.S.) hydroxyethoxyl substitution of 0.3 to 1.0 and an ethoxyl substitution of 2.5 to 2.9; and (B) 1.0 to 95 weight% of an active UV radiation absorber. Optionally included are: (C) 0 to 20 weight% of a water-insoluble organic emollient compound having a water-solubility of less than 1% at 25°C; (D) 0 to 85 weight% of a volatile liquid carrier having a melting point of less than 22°C which completely evaporates from the skin 30 minutes after application; (E) 0 to 20 weight% of suspended particulate solid matter; (F) 0 to 85 weight% of liquefied propellants; (G) 0 to 15 weight% of a thickening agent; and (H) 0 to 3 weight% of a fragrance oil. The compositions are water-proof and used as oils, lotions, creams aerosols and gels and fulfil the guidelines established by the Food and Drug Administration: Federal Register Volume 43, Number 166. The compositions allows the skin to dry without tackiness.

The US Patent No. 5'372'804 (Chesebrough-Pond's) relates to cosmetic compositions comprising at least one light screening agent wherein the light screening agent is carried in or on polymer latex particles. The polymer latex particles effect a good deposition of the light screening agents onto hair or skin. Specifically disclosed light screening agents include benzophenone compounds, dibenzoyl methane derivatives and cinnamate derivatives such as 2-ethylhexyl-p-methoxycinnamate. (PARSOL® MCX). The polymer particles employed may be of any polymeric material which is a good film former. The polymer particles may be substantially solid or may be porous and have a particle size of about 10-1000 nm. In order to get polymers having good film forming characteristics a low glass transition temperature in the region of the temperature of normal use of the cosmetic composition, approximately 30°C is required.

The above mentioned US Patent only teaches that light screening agents can be incorporated into a matrix achieving the advantage of enhanced deposition. Said US Patent does not address to the problem of photostability, which problem is given due to an interaction of 4-tert. butyl-4'-methoxydibenzoyl methane (PARSOL® 1789) with other light screening agents under light especially with 2-ethylhexyl-p-methoxycinnamate (PARSOL® MCX).

Cosmetic light screening compositions based on dibenzoyl methane derivatives as UV-A screening agent and photostabilized with 3,3-diphenylacrylate derivatives are described in the European Patent Publication EP-0 514 491 B1 and in the European Publication EP 0 780 119 A1. However, this type of stabilization does not prevent the photochemical interaction between cinnamate derivatives such as 2-ethylhexyl-p-methoxycinnamate and dibenzoyl methane derivatives such as 4-tert. butyl-4'-methoxydibenzoyl methane.

It has now been found that the photostability of light screening compositions containing a dibenzoyl methane UV-A screening agent and a p-methoxycinnamate UV-B screening agent is improved if one of said light screening agents is incorporated into a polymer latex.

Accordingly, in one aspect the invention is concerned with a method of photostabilizing mixtures of a dibenzoylmethane UV-A screening agent and a p-methoxycinnamate UV-B screening agent in a light screening composition which method comprises incorporating one of said light screening agents into a polymer latex.

In still another aspect the present invention is concerned with a light screening composition containing, based on the total weight of the composition, about 0.5 wt % to about 5 wt % of a dibenzoyl methane UV-A screening agent incorporated into a polymer latex,
about 1 wt % to about 15 wt % of a p-methoxycinnamate UV-B screening agent; and optionally other conventional UV-A and UV-B screening agents.

In yet another aspect the present invention is concerned with a light screening composition containing, based on the total weight of the composition,
about 1 wt % to about 15 wt % of p-methoxycinnamate UV-B screening agent incorporated into a polymer latex;
about 0.5 wt % to about 5 wt % of a dibenzoyl methane UV-A screening agent; and
optionally other conventional UV-A and UV-B screening agents.

As far as the dibenzoyl methane UV-A screening agent is concerned, the preferred compound is 4-tert. butyl-4'-methoxydibenzoyl methane sold under the tradename PARSOL® 1789.

Other suitable compounds of this particular type are: 2-methyldibenzoyl methane, 4-methyl-dibenzoyl methane, 4-isopropyldibenzoyl methane, 4-tert-butyldibenzoyl methane, 2,4-dimethyldibenzoyl methane, 2,5-dimethyldibenzoyl methane, 4,4'-diisopropyldibenzoyl methane, 2-methyl-5-isopropyl-4'-methoxydibenzoyl methane, 2-methyl-5-tert-butyl-4'-methoxydibenzoyl methane, 2,4-dimethyl-4'-methoxydibenzoyl methane and 2,6-dimethyl-4-tert-butyl-4'-methoxydibenzoyl methane.

As used herein the term p-methoxycinnamate UV-B screening agent refers to derivatives such as 2-ethoxyethyl-p-methoxycinnamate, 2-ethylhexyl (or pentyl) p-methoxy-cinnamate, potassium-p-methoxycinnamate, sodium-p-methoxycinnamate, ammonium-p-methoxycinnamate, salts of primary, secondary or tertiary amines of p-methoxycinnamic acid like mono-, di-, tri-ethanol amine salt and the like. Preferred is 2-ethylhexyl-p-methoxycinnamate sold under the tradename PARSOL® MCX.

As used herein the term "polymer latex" refers to a stable colloidal dispersion of polymer particles in an aqueous or an aqueous based phase including polymers and/or copolymers of unifunctional monomers and/or multifunctional monomers. The dibenzoyl methane UV-A screening agent or the p-methoxycinnamate UV-B screening agent is incorporated into the polymer matrix.

As used herein the term "unifunctional monomers" includes C₁-C₈₋alkyl(meth)acrylate, such as 2-ethyl-hexylacrylate, C₁-C₈-alkyl(meth) acrylic acid, acrylic acid, styrene, ethylene, propylene, butylene, butadiene, isoprene, isobornylmethacrylate (IBOMA), trifluoroethylmethacrylate, perfluoralkyl-2-ethylacrylate and the like.

As used herein the term "multifunctional monomers" includes allylmethacrylate (ALMA), ethyleneglycol dimethacrylate (EGDMA) and the like.

A preferred polymer latex is a stable colloidal dispersion of copolymer particles of methylmethacrylate (MMA) and acrylic acid (AA) crosslinked with allylmethacrylate (ALMA) and ethyleneglycol dimethacrylate (EGDMA) or containing isobornylmethacrylate (IBOMA) crosslinked with allylmethacrylate (ALMA).

The polymer particles can have a matrix structure within which the dibenzoyl methane UV-A screening agent or the p-methoxycinnamate UV-B screening agent is homogeneously distributed over the whole volume of the polymer particles, or the polymer particles can have a reservoir structure.

As used herein the term "reservoir structure" refers to particles having a polymer core surrounded by a polymer shell whereas the core contains the dibenzoyl methane UV-A screening agent or the p-methoxycinnamate UV-B screening agent.

In one aspect the core and the shell portions of the core/shell polymer particles may have the same chemical composition with regard to the monomers used. Suitable monomers used are unifunctional and multifunctional monomers as listed above.

In another aspect the core and shell portions of the core/shell polymer particles may differ in their chemical composition. In this case the monomers used in the core portion of the core/shell particles may also include the unifunctional and multifunctional monomers as listed above. The monomers used in the shell portion of the core/shell particles suitably include hydrophilic or fluorinated monomers.

Preferred core/shell particles include particles wherein
a) the core consists of a polymer and/or a copolymer of methylmethacrylate (MMA) and acrylic acid (AA) crosslinked with allylmethacrylate (ALMA) and ethyleneglycol dimethacrylate (EGDMA) or containing isobornylmethacrylate (IBOMA) crosslinked with allylmethacrylate (ALMA); and
b) the shell consists of hydrophilic polymer or fluorinated polymer chains.

Hydrophilic polymer shells are obtainable by copolymerization of monomers containing carboxylic acid groups such as acrylic acid, methacrylic acid, monomers bearing hydroxyl groups such as e.g. hydroxyC₁-C₆-alkyl(meth)acrylate, (HEMA) and acrylamides (AAm). A suitable hydroxy C₁-C₆-alkyl(meth)acrylate is hydroxyethylmethacrylate.

Fluorinated polymer shells are obtainable by copolymerization of acrylic esters such as e.g. methylmethacrylate (MMA) or isobornylmethacrylate (IBOMA) with fluorinated monomers such as e.g. trifluorethylmethacrylate (TRIFEMA) or perfluoroalkyl-2-ethylacrylate (PF2EA).

The core can be surrounded by one shell or by two or more shells, whereby the first shell surrounding the core and the following shell may be formed of the same polymer or of different polymers. E. g. the first shell may be formed of a hydrophilic polymer and the following shell may be formed of a fluorinated polymer and vice versa.

If core and shell are not formed of the same polymer particles a two component core-shell polymer is obtained having various morphological structures as described by Chen et al. Macromolecules 1991, 24, 3779-3787.

The polymer particles as defined above have a glass transition temperature between about 50°C and about 100°C. The glass transition temperature can be adjusted to a given value by choosing the initial content of each monomer and by crosslinking with the above mentioned multifunctional monomers. Generally, a higher content of cross-linking agent increases the glass transition temperature, while increased alkyl chain lenghts in acrylate monomers result in lower glass transition temperatures of the polymer latex. Further, the glass transition temperature of the polymer latex will be lowered by increased levels of the incorporated screening agent, e.g., PARSOL® 1789.

The glass transition temperature of the polymer used for the core portion may be the same or different compared to the glass transition temperature of the polymer used for the shell portion.

The polymer latex containing polymer particles having the dibenzoyl methane UV-A screening agent or the p-methoxycinnamate UV-B screening agent incorporated into the matrix can be produced by emulsion polymerization including a crosslinking procedure. Thus, polymer particles having a glass transition temperature > 50°C , preferably > 70°C are obtained.

The glass transition temperature is an important parameter since the polymer particles must be in a glassy state at storage temperature to impart a high stability and tightness to the system.

The preparation of latex polymers by emulsion polymerization has long been known. For example, U.S. Pat. No. 5,189,107 discloses that latex polymers having uniform particle size can be obtained by using latex seeds in the polymerization reaction to better control the particle size distribution.

Thus, the invention also comprises a process for the preparation of polymer particles having matrix structure within which a dibenzoyl methane UV-A-screening agent or a p-methoxycinnamate UV-B screening agent is homogeneously distributed over the whole volume of the particles, which process comprises
a) dissolving the dibenzoyl methane UV-A screening agent or the p-methoxycinnamate UV-B screening agent in a blend of monomers;
b) pre-emulsifying the solution of step a) in an aqueous solution containing an emulsifier;
c1) continuously introducing the pre-emulsion of step b) into a reactor containing an aqueous initiator solution or
c2) introducing a small amount of the pre-emulsion of step b) into a reactor containing an aqueous initiator solution thus, obtaining seed polymer particles and then continuously introducing the remaining pre-emulsion.

The temperature in the reactor of step c1 or c2 is from about 70°C to about 90°C preferably about 80°C.

The latex obtained by the above described emulsion polymerization process consists of a colloidal dispersion of polymer particles having a matrix structure, within which the dibenzoyl methane UV-A screening agent or the p-methoxycinnamate UV-B screening agent is homogeneously distributed over the whole volume of the particles.

A latex containing polymer particles having a reservoir structure can be prepared by a two step emulsion polymerization process comprising a first step polymerization to obtain the core polymer particles and a second step polymerization to obtain polymer particles having at least one shell surrounding the core.

The first polymerization step can be carried out as described above in steps a), b), c1) and c2).

The second polymerization step comprises another emulsion polymerization which is carried out according to steps b), c1) and c2) in the presence of the latex prepared in the first polymerization step and in the absence of light screening agents, using the same or different monomers as used in the first step.

In one embodiment of the invention the second polymerization step comprises adding monomers containing carboxylic acid groups such as acrylic acid (AA), methacrylic acid (MAA), monomers bearing hydroxyl groups such as e.g. hydroxy C₁-C₆₋alkyl(meth)acrylate, (HEMA) and acrylamides (AMD).

In another embodiment of the invention the second polymerization step comprises adding monomers containing acrylic esters such as e.g. methylmethacrylate (MMA) or isobornylmethacrylate (IBOMA) and fluorinated monomers such as e.g. trifluorethymethacrylate (TRIFEMA) or perfluoroalkyl-2-ethylacrylate (PF2EA).

The core/shell particles of the present invention can also be prepared by an inverted core/shell polymerization process, in which the shell portion is prepared first, followed by polymerization of the core monomer in the presence of the shell materials.

The weight ratio of the core portion to shell portion is suitably from about 1 to 10.

Suitable emulsifiers are anionic and nonionic surfactants, preferably anionic surfactants.

Conventional anionic surfactants such as e.g. alkyl sulfates, alkyl ether sulfates, alkyl succinates, alkyl sulfosuccinates, alkyl phosphates, alkyl ether phosphates, alkyl ether carboxylates, especially their sodium, magnesium, ammonium and mono-, di- and triethanolamine salts can be used. The alkyl groups generally contain from 8 to 18 carbon atoms and may be unsaturated.

Suitable anionic surfactants are also sorbitan derivatives sold under the tradename TWEEN by ICI Americas Incorporated, Wilmington. Preferred are polyoxyethylene-sorbitan-fatty acid esters such as e.g. polyoxyethylene (20) sorbitan monolaurate which is commercialized under the tradename TWEEN 20 (ICI Chemicals).

Other suitable and preferred surfactants are ABEX® 3594 (Rhone-Poulenc) or DOWFAX® 8390 (Dow Chemical).

As used herein the term "initiator solution" refers to an aqueous solution of peroxides, perphosphates, percarbonates, persulfates, organic peroxides and salts thereof. Preferred is ammonium persulfate.

The polymer particles have a particle size of about 100 nm to about 500 nm, preferably about 100 nm to about 400 nm, more preferably about 250 nm to about 350nm.

Where convenient the light screening composition of the present invention may further include other conventional UV-A and UV-B screening agents.

The term "conventional UV-B screening agents", i.e. substances having absorption maxima between about 290 and 320 nm, refers to the following UV-B screening agents:
--- Acrylates such as 2-ethylhexyl 2-cyano-3,3-diphenylacrylate (octocrylene, PARSOL® 340), ethyl 2-cyano-3,3-diphenylacrylate and the like;
--- Camphor derivatives such as methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
--- Organosiloxane compounds containing benzomalonate groups as described in the European Patent Publications EP 0358584 B1, EP 0538431 B1 and the European Publications EP 0709080 A1 and EP 0897716 A2.
--- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
--- Imidazole derivatives such as e.g. 2-phenyl benzimidazole sulfonic acid and its salts (PARSOL® HS). Salts of 2-phenyl-benzimidazole sulfonic acid are e.g. alkali salts such as sodium- or potassium salts, ammonium salts, morpholine salts, salts of primary, sec. and tert. amines like monoethanolamine salts, diethanolamine salts and the like.
--- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
--- Triazone derivatives such as octyl triazone (UVINUL T-150), dioctyl butamido triazone (UVASORB HEB) and the like
--- The term "conventional UV-A screening agents", i.e. substances having absorption maxima between about 320 and 400 nm, refers to the following UV-A screening agents:

--- Dibenzoyl methane derivatives such as 4-tert. butyl-4'-methoxydibenzoylmethane (PARSOL® 1789), dimethoxydibenzoyl methane, isopropyldibenzoyl methane and the like;
--- Benzotriazole derivatives such as 2-2'methylene-bis-(6-(2H-benzotriazole-2-yl)-4-(1,1,3,3,-tetramethylbutyl)-phenol (TINOSORB M) and the like;
--- Triazine derivatives such as 2,2'-[6-(4-methoxyphenyl)-1,3,5-triazine-2,4-diyl]bis[5-[2-ethylhexyl) oxy]-; available under the tradename TINOSORB S from Ciba Speciality Chemicals Holding Switzerland.
--- Pigments such as microparticulated ZnO and the like. The term "microparticulated" refers to a particle size from about 5 nm to about 200 nm, particularly from about 15 nm to about 100 nm. The ZnO particles may also be coated by metal oxides such as e.g. aluminum or zirconium oxides or by organic coatings such as e.g. polyols, methicone, aluminum stearate, alkyl silane. Such coatings are well known in the art.

Suitable organosiloxane compounds are those described in general in the European Patent EP 0538431 B1, namely compounds of the general formula I, 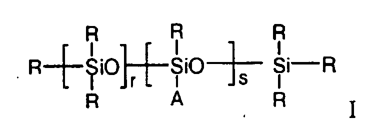 wherein
- R: signifies C₁₋₆alkyl or phenyl;
- A: signifies a group of the formula IIa and/or IIb; 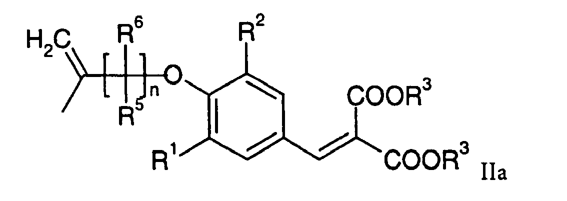 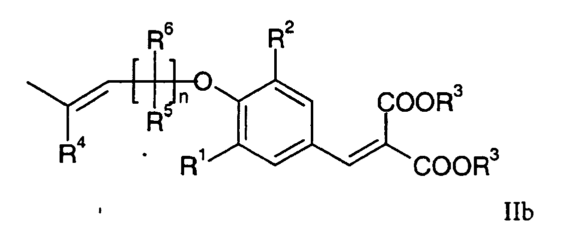
wherein
- R¹ and R²: each independently signify hydrogen, hydroxy, C₁₋₆-alkyl or C₁₋₆-alkoxy;
- R³: signifies C₁₋₆-alkyl;
- R⁴: signifies hydrogen or C₁₋₆-alkyl;
- R⁵ and R⁶: each independently signify hydrogen or C₁₋₆-alkyl;
- r: has a value of from 0 to 250;
- s: has a value of from 1 to 20;
- r +s: has a value of at least 3;
- n: has a value from 1 to 6;

The term "C₁₋₆-alkyl" refers to groups such as methyl, ethyl, propyl, isopropyl, butyl, sec. butyl, isobutyl, pentyl and neopentyl.

The term "C₁₋₆-alkoxy" refers to the corresponding alkoxy groups.

The residues R are preferably methyl.

The residues R¹ and R² are preferably hydrogen, methoxy or ethoxy, more preferably hydrogen, or one of R¹, and R² is hydrogen and the other is methyl, methoxy or ethoxy.

The residues R³ are preferably methyl or ethyl, more preferably ethyl.

Preferably, R⁴ is hydrogen or methyl, R⁵ and R⁶ are hydrogen and n is 1.

Among the above described organosiloxanes the following organosiloxane compound of the general formula I described in the European Patent Publication EP 0709080 A1 and hereinafter referred to as "Polysiloxane A" is preferred. Polysiloxane A is a compound of the above formula I, wherein
- R: signifies methyl;
- A: signifies a group of the formula IIaa and/or IIbb;

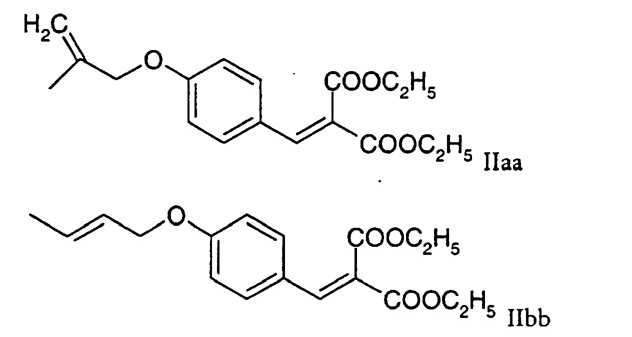

- r: is a statistical mean of about 4;
- s: is a statistical mean of about 60.

In case A signifies a group of the formula (IIa and IIb) or of the formula (IIaa and IIbb) respectively, the ratio of polysiloxane units having a chromophor residue A of the formula IIa or IIaa respectively, to those having a chromophor residue A of the formula IIb or IIbb respectively, is not critical.

Other suitable organosilioxane compounds are those described in the European Patent EP 0358584 B1, namely e.g. compounds of the general formula I wherein
- R: signifies methyl;
- A: signifies a group of the formula IIc 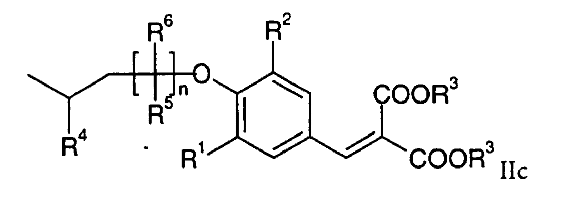
wherein R¹-R⁶ and n are as described above.

The preparation of novel light screening compositions, especially of preparations for skin protection and, respectively, sunscreen preparations for everyday cosmetics is well known to the skilled artisan in this field and comprises incorporating the polymer latex containing the dibenzoyl methane UV-A screening agent or the p-methoxycinnamate UV-B screening agent optionally together with other conventional UV-B screening agents and/or conventional UV-A screening agents as described above in a cosmetic base which is usual for light screening agents.

In a cosmetic composition of the invention the polymer latex particles act as carrier for the dibenzoyl methane UV-A screening agent or for the p-methoxycinnamate UV-B screening agent.

The solid content of polymer particles in the latex is suitably from about 10 wt % to about 50 wt %.

The latex contains the dibenzoyl methane UV-A screening agent suitably in an amount of from about 5 wt % to about 30 wt %, preferably from about 5 wt % to about 15 wt %, more preferably from about 6 wt % to about 10 wt %or the latex contains the p-methoxycinnamate UV-B screening agent in an amount of from about 1 wt % to about 15 wt %, preferably from about 2 wt % to about 8 wt %, more preferably about 5 wt %.

The amount of conventional UV-B screening agents and/or conventional UV-A screening agents is not critical. Suitable amounts are e.g.
- "Polysiloxane A":: about 0.5 to about 15wt%,
- PARSOL® 340:: about 0.5 to about lowt%,
- PARSOL® 5000:: about 0.5 to about 4wt%,
- PARSOL® HS: about 0.5 to about 10 wt %
- TINOSORB M :: about 0.5 to about 10wt%,
- TiO₂:: about 0.5 to about 25wt%,
- ZnO:: about 0.5 to about 20wt%.

The light screening compositions of the present invention can be used as cosmetic or pharmaceutical formulations. The term "cosmetic" as used herein denotes topical formulations that are intended for the maintainance, improvement or restoration of skin and hair. The term "pharmaceutical" as used herein denotes topical formulations that are intended for therapy and prophylaxis of diseases and includes formulations that contain active ingredients which exert a medicinal effect.

Suitably the compositions of this invention take the form of a lotion, a gel, a solid stick, an emulsion, e.g. cream, milk or of a vesicular dispersion of ionic or nonionic amphiphilic lipids, an aerosol, a spray, a foam, a powder, a shampoo, a hair conditioner or lacquer or a make-up, etc., see also, Sunscreens, Development, Evaluation and Regulatory Aspects, ed. N.Y. Lowe, N.A. Shaath, Marcel Dekker, Inc. New York and Basel, 1990.

The usual excipients and auxiliary agents known to the skilled practitioner can be used for the preparation of these forms, e.g. oils, waxes, alcohols, polyols, etc., particularly fatty acids, esters, fatty alcohols, but also ethanol, isopropanol, propylene glycol, glycerine, etc.

The formulations may contain further adjuvants, e.g. further solvents, thickeners, emollients, emulsifiers, humectants, tensides, preservatives, antifoams, fragrances, oils, waxes, lower polyols and monohydric alcohols, propellants, silicones, colourings and pigments, etc.

For protection of the hair, the suitable formulations are shampoos, conditioners, lotions, gels, emulsions, dispersions, lacquers, etc.

The preparation of all these formulations is well known to the skilled artisan in this field.

The following examples explain the invention in more detail but do not limit its scope in any manner.

### Example 1

### Determination of the glass transition temperature

Polymer film was prepared from the latex. The latex was dried and water evaporation gave rise to coalescence of the loaded polymer particles and formation of a cracked film. The glass transition temperature was measured by Differential Scanning Calorimetry (DSC) performed with a DSC Mettler TC11 equipment. Film samples were scanned in sealed aluminum pans in the 30°C/130°C temperature range, at a heating rate of 10°C/min.

### Example 2

### Determination of the particle loading

The quantity of dibenzoyl methane UV-A screening agent (loading) incorporated into the polymer particles was determined as follows:

The measurement of PARSOL® 1789 content within the particles (particle loading) was performed using an UV spectrometer working at a wavelength of 357 nm. A known amount of polymer film was introduced in a flask containing tetrahydrofurane (THF). This solvent swelled the latex microparticles and solubilized the encapsulated PARSOL® 1789. The absorption of PARSOL® 1789 was measured and compared with reference solution samples (calibration curve) to determine the actual PARSOL® content in the polymer film. The incorporation rate is defined as the ratio of the PARSOL® 1789 weight incorporated into the polymer particles (measured using UV-technique) over the polymer weight. The encapsulation yield is defined as the ratio of the PARSOL® 1789 weight incorporated into the polymer particles (measured using UV-technique) over the total weight of PARSOL® 1789 introduced.

### Example 3

### Determination of the photostabilizing effect

The photostabilizing effect was measured by the hereafter described method. The amount of latex necessary to get the equivalent of 1wt % PARSOL® 1789 was calculated from the solid content and the PARSOL® 1789 loading measured, 2wt % of PARSOL® MCX was added, 30% TWEEN 20 was added and finally completed to 100 wt % with distilled water.

20 mg of the above prepared solution were spread on a glass plate (10 cm²). After a drying time of 30 minutes, three identical samples were irradiated with 10 MED. The samples were then immersed in THF and placed in an ultrasound for 15 minutes. The THF of the three samples together was evaporated and 2 ml of methanol was added to the evaporate. The sample was finally filtered and analyzed by HPLC.

### Example 4

### Preparation of a latex containing EGDMA

### Step a) Solution of PARSOL® 1789

A solution of 58.9 g of PARSOL® 1789 was prepared in the blend of the following monomers: 303.9 g MMA, 9.4 g AA, 4.7 g ALMA, 15.7g EGDMA.

### Step b) Pre-emulsion

In a polyethylene vessel, 120.1 g of demineralized water were mixed with 5.9 g of an aqueous solution of ABEX ® 3594 surfactant (36% solid content) under gentle stirring. The PARSOL® 1789 solution of step a) was continuously added to the aqueous mixture under stirring (8000 rpm) during 30 minutes.

### Step c2) Polymerization

3 wt % of the pre-emulsion and a solution of ammonium persulfate were fed into the water containing reactor and heated at 80°C. After 15 minutes the remaining pre-emulsion was continuously added for 3 hours and 30 minutes at 80°C. Then the latex was cured at 80°C for 120 minutes. The reactor temperature was decreased to 60°C and a redox system ( tert. butyl hydroperoxyde/Na₂S₂O5) was fed into the reactor to eliminate residual monomers. The temperature was maintained at 60°C for 30 minutes. Finally the latex was cooled to room temperature, filtered through a 50 µm sieve and neutralized to pH 7.3 with NaOH 20wt %. The solid content of the latex was 46.1wt%. The particle size of the polymer particles in the latex was about 260 nm. The glass transition temperature was 83.5 °C.

The PARSOL® 1789 content was 12.9 wt % in the solid part of the latex and 6 wt % in the latex.

### Example 5

### Preparation of a latex containing IBOMA

### Step a) Solution of PARSOL® 1789

A solution of 58.9 g of PARSOL® 1789 was prepared in the blend of the following monomers: 249.5 g MMA, 9.7 g AA, 9.7 g ALMA, 64.8 g IBOMA.

### Step b) Pre-emulsion

In a polyethylene vessel, 132.8 g of demineralized water were mixed with 5.9 g of an aqueous solution of ABEX ® 3594 surfactant (36% solid content) under gentle stirring. The PARSOL® 1789 solution of step a) was continuously added to the aqueous mixture under stirring (8000 rpm) during 30 minutes.

### Step c2) Polymerization

3 wt % of the pre-emulsion and initiator solution were charged into the water containing reactor and heated at 80°C. After 15 minutes the remaining pre-emulsion was continuously added for 3 hours and 30 minutes at 80°C. Then the latex was cured at 80°C for 120 minutes. The reactor temperature was decreased to 60°C and a redox system ( tert. butyl hydroperoxyde/Na₂S₂O₅) was fed into the reactor to eliminate residual monomers. The temperature was maintained at 60°C for 30 minutes. Finally the latex was cooled to room temperature, filtered through a 50 µm sieve and neutralized to pH 7.3 with NaOH 20wt %. The solid content of the latex was 45.2 wt %. The particle size of the polymer particles in the latex was about 303 nm. The glass transition temperature was 90.0 °C.

The PARSOL® 1789 content was 13.3 wt % in the solid part of the latex and 6 wt % in the latex.

### Example 6

In analogy to Example 4, compositions of modified polymer matrices including functional hydrophilic monomers in the polymerisation step were prepared. The compositions and results are shown in Table I below:

**Table I**

| No. | % MMA | % AA | % ALMA | % EGDMA | hydrophilic monomer % | Tg °C | Parsol®1789 measured % | solids measured % | mean diameter nm |
|---|---|---|---|---|---|---|---|---|---|
| i | 85 | 3 | 1.5 | 5 | HEMA - 5.5 | 83.8 | 13.1 | 46.6 | 275 |
| ii | 88.5 | 3 | 1.5 | 5 | HEMA - 2 | 93.5 | 15 | 43.9 | 262 |
| iii | 88.5 | 3 | 1.5 | 5 | HPMA - 2 | 89.8 | 12.8 | 47.2 | 269 |
| iv | 88.5 | 3 | 1.5 | 5 | AMPS - 2 | 85.6 | 12.4 | 46.2 | 287 |
| v | 88.5 | 3 | 1.5 | 5 | AA - 5 | 113.3 | 12.4 | 44.5 | 264 |
| vi | 88.5 | 3 | 1.5 | 5 | AMD- 1 | 89 | 12.5 | 45.5 | 256 |

### Example 7

In analogy to the process of Example 4, but adding a hydrophilic monomer (methacrylic or acrylic acid) after the introduction of 80 % of the pre-emulsion the compositions as in Table II were prepared:

**Table II**

| No. | % MMA | % AA | % ALMA | % EGDMA | hydrophilic monomer % | Tg °C | Parsol® 1789 measured % | solids measured % | mean diameter nm |
|---|---|---|---|---|---|---|---|---|---|
| vii | 86.5 | 3 | 2 | 5 | AA - 3.5 | 79.5 | 14.3 | 45.3 | 336 |
| viii | 86.5 | 3 | 1.5 | 5 | AMA - 3.5 | 86.5 | 12 | 45.9 | 303 |

### Example 8

Core-shell nanoparticles were prepared by a two-step emulsion polymerization process. A dispersion of nanoparticles (prepared in analogy to Example 4 using DOWFAX 8390 as the surfactant in the pre-emulsion) is used as a seed to polymerize a polymer shell around the core particles so as to produce a polymer membrane free of screening agent and including hydrophilic monomers. Several blends of monomers were used as summarized in Table III below:

**Table III**

| Blend | MMA | MAA | HEMA | EGDMA | ALMA |
|---|---|---|---|---|---|
| 1 | 41% | 15% | 40% | 2% | 2% |
| 2 | 61% | 10% | 25% | 2% | 2% |
| 3 | 73.5% | 7.5% | 15% | 2% | 2% |
| 4 | 81% | 15% | - | 2% | 2% |

The polymerization process of the shell is performed as described without dilution of the seed latex. However, the same amount of water as the amount of monomers used in the preparation of the polymer shell is added to the polymerization reaction to form the shell. Therefore, the seed latex and the final core-shell latex display the same solid content. The compositions and the results obtained are given in Table IV:

**Table IV**

| Sample | Seed Latex % SC* %PARSOL® 1789 | | Monomer Blend No. % added | | Tg °C | PARSOL®1789 measured (%) | Solid Content measured (%) | Mean diameter (nm) |
|---|---|---|---|---|---|---|---|---|
| ix | 30 | 20 | 1 | 10 | 75.1 | 15.4 | 30.3 | 137 |
| | | | | | 102.1 | | | |
| x | 40 | 15 | 2 | 20 | 88.9 | 9.4 | 37.5 | 281 |
| | | | | | 140.9 | | | |
| xi | 40 | 15 | 4 | 20 | 95.4 | 9.9 | 38.9 | 367 |
| | | | | | 137.4 | | | |
| xii | 50 | 15 | 3 | 20 | 95.8 | 10.6 | 47.2 | 315 |
| | | | | | 140.4 | | | |
| xiii | 50 | 20 | 3 | 20 | 87.6 | 13 | 46.4 | 318 |
| | | | | | 135.2 | | | |
| xiv | 50 | 20 | 4 | 20 | 89.9 | 14 | 46.8 | 314 |
| | | | | | 110.5 | | | |

| | | | | | | | | |
|---|---|---|---|---|---|---|---|---|
| SC* solid content | | | | | | | | |

These latices exhibit two glass transition temperatures Tg, the first Tg pertaining to the core polymer and the second Tg pertaining to the shell polymer.

### Example 9

A latex wherein PARSOL® MCX is incorporated into the polymer particles was prepared in an analogous manner as described in Examples 4 and 5.

The following Examples 10 and 11 refer to sunscreen compositions. The abbreviations and trade names selected have the following significance:
- ARLACEL P135: PEG-30 dipolyhydroxystearate sold by ICI
- ARLAMOL E: POP-(15)-stearyl alcohol sold by ICI
- ARLAMOL HD: Heptamethylnonane sold by ICI
- BHT: Butylhydroxytoluol (2,6 di-tert butyl-4-methyl phenol)
- BRIJ 72: POE-(2)-stearyl alcohol sold by ICI
- BRIJ 721: POE-(21)-stearyl alcohol sold by ICI
- EDETA BD: Disodium EDTA sold by BASF
- LANETTE O: Cetearyl alcohol sold by Henkel
- PARSOL® 340: Octocrylene sold by Roche
- PARSOL® 1789: Butyl methoxydibenzoylmethane sold by Roche
- PARSOL® 5000: Methyl benzylidene camphor sold by Roche
- PARSOL® HS: Phenyl benzimidazole sulfonic acid sold by Roche
- PARSOL® MCX: 2-Ethylhexyl-p-methoxycinnamate sold by Roche
- PHENONIP: Phenoxyethanol (and) methylparaben (and) butylparaben (and) ethylparaben (and) propylparaben sold by NIPA
- Propylene glycol: 1,2 propanediol sold by BASF
- SILBIONE oil 70047 V20: Cyclomethicone sold by Rhône-Poulenc
- SILICONE 1401 Fluid: Cyclomethicone & dimethiconol sold by Dow Corning
- SILICONE 5225 C: Cyclopentasiloxane & dimethicone copolyol sold by
- Formulation Aid: Dow Corning
- SILICONE DC 344: Cyclomethicone sold by Dow Corning
- SILICONE DC 5200: Dimethicone copolyol sold by Dow Corning
- UMORDANT P: Na-Lactate & Na-PCA & urea & hydrolyzed vegetable protein & Aspa.

### Example 10

Preparation of a sunscreen O/W lotion containing 23 wt % encapsulated PARSOL® 1789 prepared according to Ex. 4.

| Ingredients | % w/w |
|---|---|
| ARLAMOL E | 5.00 |
| ARLAMOL HD | 5.00 |
| BRIJ 72 | 3.00 |
| BRIJ 721 | 2.00 |
| ARLACEL P135 | 0.50 |
| LANETTE O | 5.00 |
| Stearic Acid | 1.50 |
| SILBIONE Oil 70047V20 | 1.00 |
| BHT | 0.10 |
| PHENONIP | 0.60 |
| Deionized Water qsp to | 100.00 |
| Xanthan Gum 1% solution | 6.00 |
| Propylene Glycol | 4.00 |
| UMORDANT P | 1.00 |
| PARSOL® 1789 encapsulated | 23.00 |
| PARSOL® MCX | 5.00 |

The organic phase containing the UV-filters was heated to 75°C, then the pre-heated aqueous phase (75° C) was added while stirring. The resulting emulsion was cooled to ambient temperature.

### Example 11

Preparation of a sunscreen O/W lotion containing 23 wt % encapsulated PARSOL® 1789 prepared according to Ex. 4.

| Ingredients | %w/w |
|---|---|
| SILICONE 1401 Substantivity Aid Fluid | 10.00 |
| SILICONE 3225C Formulation Aid | 10.00 |
| SILICONE DC 344 | 10.00 |
| SILICONE DC 5200 | 2.00 |
| EDETA BD | 0.10 |
| PHENONIP | 0.60 |
| PARSOL® HS | 3.00 |
| Glycerol | 5.00 |
| Deionized Water qsp to | 100.00 |
| PARSOL® 1789 encapsulated | 23.00 |
| Sodium Hydroxyde 10% qsp | pH 7 |

The organic phase containing the UV-filters was heated to 75°C, then the pre-heated aqueous phase (75° C) was added while stirring. The resulting emulsion was cooled to ambient temperature.

## Claims

1. A photostable cosmetic or pharmaceutical light screening composition containing a dibenzoyl methane UV-A screening agent and a p-methoxycinnamate UV-B screening agent wherein one of the screening agents is incorporated into a polymer matrix.

2. A light screening composition as in claim 1 containing 0.5 wt % to 5 wt % of a dibenzoyl methane UV-A screening agent incorporated into a polymer latex;
1 wt % to 15 wt % of a p-methoxycinnamate UV-B screening agent; and optionally other conventional UV-A and UV-B screening agents.

3. A light screening composition as in claim 1 containing 1 wt % to 15 wt % of a p-methoxycinnamate UV-B screening agent incorporated into a polymer latex;
0.5 wt % to 5 wt % of a dibenzoyl methane UV-A screening agent; and optionally other conventional UV-A and UV-B screening agents.

4. A light screening composition according to any one of claims 1 to 3, wherein the dibenzoyl methane UV-A screening agent is 4-tert. butyl-4'-methoxydibenzoyl methane and the p-methoxycinnamate UV-B screening agent is 2-ethylhexyl p-methoxycinnamate.

5. A light screening composition according to any one of claims 1 to 4, wherein the polymer latex is a stable colloidal dispersion of polymer particles in an aqueous.or an aqueous based phase including polymers and/or copolymers of unifunctional monomers and/or multifunctional monomers.

6. A light screening composition according to claim 5, wherein the unifunctional monomer is selected from C₁-C₈-alkyl(meth)acrylate, C₁-C₈-alkyl(meth) acrylic acid, acrylic acid, styrene, ethylene, propylene, butylene, butadiene, isoprene, isobornylmethacrylate (IBOMA), trifluoroethylmethacrylate or perfluoralkyl-2-ethylacrylate.

7. A light screening composition according to claim 5, wherein the multifunctional monomer is selected from allylmethacrylate (ALMA) or ethyleneglycol dimethacrylate (EGDMA).

8. A light screening composition according to any one of claims 5 to 7, wherein the polymer particles have a glass transition temperature between 50°C and 100°C.

9. A light screening composition according to any one of claims 1 to 8, wherein the polymer latex is a stable colloidal dispersion of copolymer particles of methylmethacrylate (MMA) and acrylic acid (AA) crosslinked with allylmethacrylate (ALMA) and ethyleneglycol dimethacrylate (EGDMA) or containing isobornylmethacrylate (IBOMA)-crosslinked with allylmethacrylate (ALMA).

10. A light screening composition according to any one of claims 5 to 9, wherein the polymer particles have a matrix structure within which the dibenzoyl methane UV-A screening agent or the p-methoxycinnamate UV-B screening agent is homogeneously distributed over the whole volume of the particles.

11. A light screening composition according to any one of claims 5 to 9, wherein the polymer particles have a polymer core surrounded by a polymer shell whereas the core contains the dibenzoyl methane UV-A screening agent or the-methoxycinnamate UV-B screening agent.

12. A light screening composition according to claim 11, wherein the core and the shell portions of the core/shell polymer particles have the same chemical composition with regard to the monomers used.

13. A light screening composition according to claim 11, wherein the core and shell portions of the core/shell particles differ in their chemical composition.

14. A light screening composition according to claim 13, wherein
a) the core consists of a polymer and/or a copolymer of methylmethacrylate (MMA) and acrylic acid (AA) crosslinked with allylmethacrylate (ALMA) and ethyleneglycol dimethacrylate (EGDMA) or containing isobornylmethacrylate (IBOMA) crosslinked with allylmethacrylate (ALMA); and
b) the shell consists of hydrophilic polymer or fluorinated polymer chains.

15. A light screening composition according to any one of claims 1-14 for use as a cosmetic or pharmaceutical for human skin or hair

16. A process for preparing the polymer latex as defined in any of claims 1 to 10, wherein one of the screening agents is incorporated into, the screening agents being dibenzoyl methane UV-A screening agent and a p-methoxycinnamate UV-B screening agent, and wherein the polymer particles have matrix structure, within which the dibenzoyl methane UV-A screening agent or the p-methoxycinnamate UV-B screening agent is homogeneously distributed over the whole volume of the particles, which process consists of
a) dissolving the dibenzoyl methane UV-A screening agent or the p-metboxycinnamate UV-B screening agent in a blend of monomers;
b) pre-emulsifying the solution of step a) in an aqueous solution containing an emulsifier;
c1) continuously introducing the pre-emulsion of step b) into a reactor containing an aqueous initiator solution or
c2) introducing a small amount of the pre-emulsion of step b) into a reactor containing an aqueous initiator solution thus, obtaining seed polymer particles and then continuously introducing the remaining pre-emulsion.

17. A process for preparing the polymer latex as defined in any of claims 1 to 9 and 11 to 14, wherein one of the screening agents is incorporated into, the screening agents being dibenzoyl methane UV-A screening agent and a p-methoxycinnamate UV-B screening agent, and wherein the polymer particles have a polymer core surrounded by a polymer shell which process consists of a two step emulsion polymerization process comprising a first step polymerization to obtain the core polymer particles and a second step polymerization to obtain polymer particles having at least one shell surrounding the core.

18. A method of photostabilizing mixtures of a dibenzoylmethane UV-A screening agent and a p-methoxycinnamate UV-B screening agent in a light screening composition which method comprises incorporating one of said light screening agents into a polymer latex.

## Patentansprüche

1. Fotostabile kosmetische oder pharmazeutische Lichtschutzzusammensetzung, die ein Dibenzoylmethan-UV-A-Schutzmittel und ein p-Methoxycinnamat-UV-B-Schutzmittel enthält, wobei eines der Schutzmittel in eine Polymermatrix eingebracht ist.

2. Lichtschutzzusammensetzung wie in Anspruch 1, enthaltend 0,5 Gew.-% bis 5 Gew.-% eines in einen Polymerlatex eingebrachten Dibenzoylmethan-UV-A-Schutzmittels, 1 Gew.-% bis 15 Gew.-% eines p-Methoxycinnamat-UV-B-Schutzmittels; und gegebenenfalls andere herkömmliche UV-A- und UV-B-Schutzmittel.

3. Lichtschutzzusammensetzung wie in Anspruch 1, enthaltend 1 Gew.-% bis 15 Gew.-% eines in einen Polymerlatex eingebrachten p-Methoxycinnamat-UV-B-Schutzmittels, 0,5 Gew.-% bis 5 Gew.-% eines Dibenzoylmethan-UV-A-Schutzmittels; und gegebenenfalls andere herkömmliche UV-A- und UV-B-Schutzmittel.

4. Lichtschutzzusammensetzung gemäß mindestens einem der Ansprüche 1 bis 3, wobei das Dibenzoylmethan-W-A-Schutzmittel 4-tert.-Butyl-4'-methoxydibenzoylmethan ist das p-Methoxycinnamat-UV-B-Schutzmittel 2-Ethylhexyl-p-methoxycinnamat ist.

5. Lichtschutzzusammensetzung gemäß mindestens einem der Ansprüche 1 bis 4, wobei der Polymerlatex eine stabile kolloidale Dispersion von Polymerteilchen in einer wässrigen oder einer auf Wasser basierenden Phase ist, die Polymere und/oder Copolymere von unifunktionellen Monomeren und/oder multifunktionellen Monomeren einschließt.

6. Lichtschutzzusammensetzung gemäß Anspruch 5, wobei das unifunktionelle Monomer aus C₁-C₈₋Alkyl (meth) acrylat, C₁-C₈-Alkyl (meth) acrylsäure, Acrylsäure, Styrol, Ethylen, Propylen, Butylen, Butadien, Isopren, Isobornylmethacrylat (IBOMA), Trifluorethylmethacrylat oder Perfluoralkyl-2-ethylacrylat gewählt ist.

7. Lichtschutzzusammensetzung gemäß Anspruch 5, wobei das multifunktionelle Monomer aus Allylmethacrylat (ALMA) oder Ethylenglycoldimethacrylat (EGDMA) gewählt ist.

8. Lichtschutzzusammensetzung gemäß mindestens einem der Ansprüche 5 bis 7, wobei die Polymerteilchen eine Glasübergangstemperatur zwischen 50 °C und 100 °C besitzen.

9. Lichtschutzzusammensetzung gemäß mindestens einem der Ansprüche 1 bis 8, wobei der Polymerlatex eine stabile kolloidale Dispersion von Copolymerteilchen aus Methylmethacrylat (MMA) und Acrylsäure (AA), vernetzt mit Allylmethacrylat (ALMA) und Ethylenglycoldimethacrylat (EGDMA), oder enthaltend Isobornylmethacrylat (IBOMA), vernetzt mit Allylmethacrylat (ALMA), ist.

10. Lichtschutzzusammensetzung gemäß mindestens einem der Ansprüche 5 bis 9, wobei die Polymerteilchen eine Matrixstruktur besitzen, innerhalb der das Dibenzoylmethan-UV-A-Schutzmittel oder das p-Methoxycinnamat-UV-B-Schutzmittel homogen über das ganze Volumen der Teilchen verteilt ist.

11. Lichtschutzzusammensetzung gemäß mindestens einem der Ansprüche 5 bis 9, wobei die Polymerteilchen einen Polymerkern besitzen, der von einer Polymerhülle umgeben ist, wobei der Kern das Dibenzoylmethan-UV-A-Schutzmittel oder das p-Methoxycinnamat-UV-B-Schutzmittel enthält.

12. Lichtschutzzusammensetzung gemäß Anspruch 11, wobei die Kern- und Hüllenbereiche der Kern/Hüllen-Polymerteilchen die gleiche chemische Zusammensetzung in Bezug auf die verwendeten Monomere besitzen.

13. Lichtschutzzusammensetzung gemäß Anspruch 11, wobei die Kern- und Hüllenbereiche der Kern/Hüllen-Teilchen sich in ihrer chemischen Zusammensetzung unterscheiden.

14. Lichtschutzzusammensetzung gemäß Anspruch 13, wobei
a) der Kern aus einem Polymer und/oder einem Copolymer von Methylmethacrylat (MMA) und Acrylsäure (AA), vernetzt mit Allylmethacrylat (ALMA) und Ethylenglycoldimethacrylat (EGDMA), oder enthaltend Isobornylmethacrylat (IBOMA), vernetzt mit Allylmethacrylat (ALMA), besteht; und
b) die Hülle aus hydrophilem Polymer oder fluorierten Polymerketten besteht.

15. Lichtschutzzusammensetzung gemäß mindestens einem der Ansprüche 1 - 14 zur Verwendung als ein Kosmetikum oder Pharmazeutikum für menschliche(s) Haut oder Haar.

16. Verfahren zur Herstellung des Polymerlatex, wie in mindestens einem der Ansprüche 1 bis 10 definiert, in welchen eines der Schutzmittel eingebracht wird, wobei die Schutzmittel Dibenzoylmethan-UV-A-Schutzmittel und ein p-Methoxycinnamat-UV-B-Schutzmittel sind, und wobei die Polymerteilchen eine Matrixstruktur aufweisen, innerhalb der das Dibenzoylmethan-UV-A-Schutzmittel oder das p-Methoxycinnamat-UV-B-Schutzmittel homogen über das ganze Volumen der Teilchen verteilt ist, wobei das Verfahren aus Folgendem besteht
a) Auflösen des Dibenzoylmethan-UV-A-Schutzmittels oder des p-Methoxycinnamat-UV-B-Schutzmittels in einer Mischung von Monomeren;
b) Voremulgierung der Lösung von Schritt a) in einer wässrigen Lösung, die ein Emulgiermittel enthält;
c1) kontinuierliches Einführen der Voremulsion von Schritt b) in einen Reaktor, der eine wässrige Initiatorlösung enthält, oder
c2) Einführen einer kleinen Menge der Voremulsion von Schritt b) in einen Reaktor, der eine wässrige Initiatorlösung enthält, wodurch Keim-Polymerteilchen erhalten werden, und anschließendes kontinuierliches Einführen der restlichen Voremulsion.

17. Verfahren zur Herstellung des Polymerlatex, wie in mindestens einem der Ansprüche 1 bis 9 und 11 bis 14 definiert, in den eines der Schutzmittel eingebracht ist, wobei die Schutzmittel Dibenzoylmethan-UV-A-Schutzmittel und ein p-Methoxycinnamat-UV-B-Schutzmittel sind, und wobei die Polymerteilchen einen Polymerkern besitzen, der von einer Polymerhülle umgeben ist, wobei das Verfahren aus einem Zwei-Schritt-Emulsionspolymerisationsverfahren besteht, umfassend eine Erst-Schritt-Polymerisation, um die Kern-Polymerteilchen zu erhalten, und eine Zweit-Schritt-Polymerisation, um Polymerteilchen, die mindestens eine den Kern umgebende Hülle aufweisen, zu erhalten.

18. Verfahren der Fotostabilisierung von Mischungen aus einem Dibenzoylmethan-UV-A-Schutzmittel und einem p-Methoxycinnamat-UV-B-Schutzmittel in einer Lichtschutzzusammensetzung, wobei das Verfahren das Einbringen von einem der Lichtschutzmittel in einen Polymerlatex umfasst.

## Revendications

1. Composition cosmétique ou pharmaceutique d'écran solaire photostable contenant un agent faisant écran aux UV-A de type dibenzoylméthane et un agent faisant écran aux UV-B de type p-méthoxycinnamate dans laquelle un des agents faisant écran est incorporé dans une matrice de polymère.

2. Composition d'écran solaire selon la revendication 1 contenant de 0,5 % en poids à 5 % en poids d'un agent faisant écran aux UV-A de type dibenzoylméthane incorporé dans un latex de polymère, de 1 % en poids à 15 % en poids d'un agent faisant écran aux UV-B de type p-méthoxycinnamate, et éventuellement d'autres agents faisant écran aux UV-A et aux UV-B classiques.

3. Composition d'écran solaire selon la revendication 1 contenant de 1 % en poids à 15 % en poids d'un agent faisant écran aux UV-B de type p-méthoxycinnamate incorporé dans un latex de polymère, de 0,5 % en poids à 5 % en poids d'un agent faisant écran aux UV-A de type dibenzoylméthane, et éventuellement d'autres agents faisant écran aux UV-A et aux UV-B classiques.

4. Composition d'écran solaire selon l'une quelconque des revendications 1 à 3, dans laquelle l'agent faisant écran aux UV-A de type dibenzoylméthane est le 4-t-butyl-4'-méthoxydibenzoylméthane et l'agent faisant écran aux UV-B de type p-méthoxycinnamate est le p-méthoxycinnamate de 2-éthylhexyle.

5. Composition d'écran solaire selon l'une quelconque des revendications 1 à 4, dans laquelle le latex de polymère est une dispersion colloïdale stable de particules de polymère dans une phase aqueuse ou une phase à base aqueuse renfermant des polymères et/ou copolymères de monomères monofonctionnels et/ou de monomères multifonctionnels.

6. Composition d'écran solaire selon la revendication 5, dans laquelle le monomère monofonctionnel est choisi parmi un (méth)acrylate d'alkyle en C₁-C₈, un acide alkyl (en C₁-C₈) (méth) acrylique, l'acide acrylique, le styrène, l'éthylène, le propylène, le butylène, le butadiène, l'isoprène, le méthacrylate d'isobornyle (IBOMA), le méthacrylate de trifluoroéthyle ou un 2-éthylacrylate de perfluoroalkyle.

7. Composition d'écran solaire selon la revendication 5, dans laquelle le monomère multifonctionnel est choisi parmi le méthacrylate d'allyle (ALMA) ou le diméthacrylate d'éthylèneglycol (EGDMA).

8. Composition d'écran solaire selon l'une quelconque des revendications 5 à 7, dans laquelle les particules de polymère ont une température de transition vitreuse comprise entre 50°C et 100°C.

9. Composition d'écran solaire selon l'une quelconque des revendications 1 à 8, dans laquelle le latex de polymère est une dispersion colloïdale stable de particules de copolymère de méthacrylate de méthyle (MMA) et d'acide acrylique (AA) réticulé avec du méthacrylate d'allyle (ALMA) et du diméthacrylate d'éthylèneglycol (EGDMA) ou contenant du méthacrylate d'isobornyle (IBOMA) réticulé avec du méthacrylate d'allyle (ALMA).

10. Composition d'écran solaire selon l'une quelconque des revendications 5 à 9, dans laquelle les particules de polymère ont une structure de matrice dans laquelle l'agent faisant écran aux UV-A de type dibenzoylméthane ou l'agent faisant écran aux UV-B de type p-méthoxycinnamate est distribué de façon homogène sur tout le volume des particules.

11. Composition d'écran solaire selon l'une quelconque des revendications 5 à 9, dans laquelle les particules de polymère ont un coeur de polymère entouré d'une coquille de polymère alors que le coeur contient l'agent faisant écran aux UV-A de type dibenzoylméthane ou l'agent faisant écran aux UV-B de type p-méthoxycinnamate.

12. Composition d'écran solaire selon la revendication 11, dans laquelle les parties coeur et coquille des particules de polymère coeur/coquille ont la même composition chimique en ce qui concerne les monomères utilisés.

13. Composition d'écran solaire selon la revendication 11, dans laquelle les parties coeur et coquille des particules coeur/coquille ont des compositions chimiques différentes.

14. Composition d'écran solaire selon la revendication 13, dans laquelle
a) le coeur est constitué d'un polymère et/ou d'un copolymère de méthacrylate de méthyle (MMA) et d'acide acrylique (AA) réticulé avec du méthacrylate d'allyle (ALMA) et du diméthacrylate d'éthylèneglycol (EGDMA) ou contenant du méthacrylate d'isobornyle (IBOMA) réticulé avec du méthacrylate d'allyle (ALMA); et
b) la coquille est constituée de chaînes de polymère hydrophile ou de polymère fluoré.

15. Composition d'écran solaire selon l'une quelconque des revendications 1-14 à utiliser comme produit cosmétique ou pharmaceutique pour la peau ou les cheveux humains.

16. Procédé de préparation du latex de polymère tel que défini dans l'une quelconque des revendications 1 à 10, dans lequel est incorporé un des agents faisant écran, les agents faisant écran étant un agent faisant écran aux UV-A de type dibenzoylméthane et un agent faisant écran aux UV-B de type p-méthoxycinnamate, et dans lequel les particules de polymère ont une structure de matrice au sein de laquelle l'agent faisant écran aux UV-A de type dibenzoylméthane ou l'agent faisant écran aux UV-B de type p-méthoxycinnamate est distribué de façon homogène sur tout le volume des particules, ce procédé consistant à
a) dissoudre l'agent faisant écran aux UV-A de type dibenzoylméthane ou l'agent faisant écran aux UV-B de type p-méthoxycinnamate dans un mélange de monomères,
b) pré-émulsionner la solution de l'étape a) dans une solution aqueuse contenant un émulsifiant,
c1) introduire en continu la pré-émulsion de l'étape b) dans un réacteur contenant une solution aqueuse d'initiateur ou
c2) introduire une petite quantité de la pré-émulsion de l'étape b) dans un réacteur contenant une solution aqueuse d'initiateur afin d'obtenir des particules de germe de polymère et introduire ensuite en continu le reste de la pré-émulsion.

17. Procédé de préparation du latex de polymère tel que défini dans l'une quelconque des revendications 1 à 9 et 11 à 14, dans lequel est incorporé un des agents faisant écran, les agents faisant écran étant un agent faisant écran aux UV-A de type dibenzoylméthane et un agent faisant écran aux UV-B de type p-méthoxycinnamate, et dans lequel les particules de polymère ont un coeur de polymère entouré d'une coquille de polymère, ce procédé consistant en un procédé de polymérisation en émulsion en deux étapes comprenant une première étape de polymérisation permettant d'obtenir les particules de polymère de coeur et une deuxième étape de polymérisation permettant d'obtenir des particules de polymère possédant au moins une coquille entourant le coeur.

18. Procédé de photostabilisation de mélanges d'un agent faisant écran aux UV-A de type dibenzoylméthane et d'un agent faisant écran aux UV-B de type p-méthoxycinnamate dans une composition d'écran solaire, ce procédé comprenant le fait d'incorporer un desdits agents écrans solaires dans un latex de polymère.
